Europäisches Patentamt

European Patent Office

Office européen des brevets

(11) Publication number: **0 489 217 A1**

## EUROPEAN PATENT APPLICATION

(21) Application number: **90830564.2**

(22) Date of filing: **05.12.90**

(51) Int. Cl.5: **A61K 37/30**, A61K 9/12, A61K 47/18

(43) Date of publication of application:
**10.06.92 Bulletin 92/24**

(84) Designated Contracting States:
**AT BE CH DE DK ES FR GB GR IT LI LU NL SE**

(71) Applicant: **DR. A. TOSI FARMACEUTICI S.R.L.**
**Corso Della Vittoria, 12/B**
**I-28100 Novara(IT)**

(72) Inventor: **Tosi, Silvana**
**Corso Della Vittoria, 12/B**
**I-28100 Novara(IT)**
Inventor: **Dondi, Giancarla**
**Corso Della Vittoria, 12/B**
**I-28100 Novara(IT)**

(74) Representative: **Bianchetti, Giuseppe**
**Studio Consulenza Brevettuale Via Rossini, 8**
**I-20122 Milan(IT)**

(54) Calcitonin compositions for intranasal administration.

(57) The invention relates to stable in the air pharmaceutical composition for the intranasal administration containing a) calcitonin dissolved in b) a citrate buffered aqueous solution at pH 4.0 stabilized by a sodium p-hydroxybenzoates mixture and containing sodium edetate. Said composition is administered in the form of a nasal spray.

The present invention relates to a set of galenic compositions containing calcitonin as the active ingredient. The compositions object of the present invention, which can be administered by the intranasal route, consist of calcitonin (preferably from salmon) dissolved in a citrate buffered solution at pH 4.0 containing a methyl and propyl p-hydroxybenzoates mixture (as sodium salts), said compositions are characterized in containing also sodium edetate (ethylenediaminetetraacetic acid tetrasodium salt).

Calcitonin, both from salmon and from other species, is biochemically defined as a long chain peptide hormon pharmacologically active in the osteoporosis therapy, in the Paget's disease, in the hypercalcemia and it acts by inhibiting physiological or pathological bony resorption. Its amino acidic nature makes it ineffective by oral administration while some drawbacks occur when calcitonin is injected, due to painful reactions in patients.

Intranasal administration of calcitonin as well as that of other polypeptide drugs, has been for a long time the object of several studies on international scientific papers and it constitutes the subject-matter of many patent specifications.

Calcitonin formulations containing hydroxybenzoates and buffers of various kind for the intranasal administration are already well-known (UK 1,354,526). It is also well-known that, during the production stage, these solutions require to be protected against oxygen both under inert gas streaming into the solutions, and by introducing an inert gas into the containers.

Sensitivity of calcitonin solutions to atmospheric oxygen is confirmed by EP-A-363876, relating to calcitonin solutions containing only sodium chloride, which are administered from specific containers filled with nitrogen.

Now it has surprisingly been found that adding sodium edetate to calcitonin aqueous solutions, preferably containing also methyl and propyl p-hydroxybenzoates, makes the solutions perfectly stable to oxygen thus allowing to operate during production without protecting with inert gases, and to pack the solutions in normal containers which in their turn require no protection with inert gases. The advantages that come from the invention, both in operative simplicity terms and in cost terms and easy storage of pharmaceutical compositions, are evident.

Therefore, the present invention provides pharmaceutical compositions containing a) calcitonin dissolved in b) a citrate buffered aqueous solution at pH 4.0, optionally added with methyl and/or propyl p-hydroxybenzoates, characterized in that they further contain c) sodium ethylenediaminetetraacetate.

Preferably, the compositions according to the invention consist of solutions containing about 100 to about 5000 I.U. of calcitonin (preferably salmon's calcitonin, i.e. salcatonin) per millilitre; a buffer formed by citric acid and sodium citrate dihydrate in such a quantity to adjust pH to 4.0; 1 to 2 mg methyl p-hydroxybenzoate and 0.1 to 0.5 mg propyl p-hydroxybenzoate (both of them as sodium salts); and 0.5 to 1.5 mg of sodium edetate (ethylenediaminetetraacetic acid tetrasodium salt).

Particularly preferred pharmaceutical compositions are those containing, per millilitre, 250 to 2000 I.U. salcatonin; 6.27 to 18.8 mg citric acid; 6.19 to 18.56 mg sodium citrate dihydrate; 0.75 to 2.25 mg methyl p-hydroxybenzoate sodium salt; 0.11 to 0.34 mg propyl p-hydroxybenzoate sodium salt; and 0.5 to 1.5 mg ethylenediaminetetraacetic acid tetrasodium salt.

A further object of the present invention is provided by a process for the preparation of said compositions, comprising 1) dissolving appropriate calcitonin amounts in a citrate buffered aqueous solution at pH 4, optionally added with methyl and propyl p-hydroxybenzoates in the abovestated ratios, and further containing an appropriate sodium edetate amount, 2) filtrating and 3) packing in appropriate containers, being all the process steps carried out without inert gases protection.

The stability of the preparations object of the invention was determined according to well-known methods, both in the presence of nitrogen atmosphere and in the lack of the same. At the end of the stability test, carried out within a 24 month interval, calcitonin content in the solution under nitrogen atmosphere is not significantly different from the one obtained for the solution without nitrogen atmosphere.

Therefore, for instance, both solutions degrade less than 10% within 2 years at 20°C. Moreover, both solutions, stored for 4 months at 5° and 20°C, undergo no significant degradation (lower than 1.5%).

A higher degradation was observed at 30°C for 3 months (about 4.5%), such value is anyway lower than the one expected for a pure aqueous solution.

Also bottles, from which half content had already been sprayed, were kept under control. Tests were performed under the same conditions reported by F.U. (Official Italian Pharmacopoeia), IX Edition, Vol II, page 1414, for calcitonin injectable preparations, except inert gas protection.

Results are reported in Table n. 1 for the intact bottle and in Table n. 2 for bottles part of the content of which had already been dispensed, to confirm pack tightness and long-term stability even after partial use.

Results confirm that there is no difference between the intact product and the used product. Results obtained after 2 years are whithin the limits established by F.U., IX Edition for injectable preparations (80-121%).

Definitely lower results were obtained on preparations obtained in the same manner but without sodium edetate, intact (Table 3) and subjected to partial dispensing (Table 4).

The Applicant carried out stability tests even against possible microbial contaminations.

The compositions were stored at 30°C for 3 months in a glass bottle. Tests were carried out according to the process by S. Urban in Acta Pharmacol. Technol., 22, 247-253, 1976. Concerning this, classical bacteria, such as E. coli ATCC 8739, Pseud. aeruginosa ATCC 9027, Staph. aureus ATCC 6538 and classical fungi like Candida albicans ATCC 10231, Aspergillus niger ATCC 16404 and Sacch. cerevisiae ATCC 9763 were used. Bacteria and fungi were added to the solution so that a cell number of about $2 \times 10^5$ organisms in the inoculated liquid was obtained. After 4 hour incubation the number of microorganisms was remarkably reduced, going to less than 0.2% and 5 weeks later no living cells were noticed in the solution. Such negativeness lasted till the end of the stability tests.

The results obtained from clinical tests showed that the composition is well tolerated, except for a slight tingling sensation in rare cases.

The following examples illustrate the invention.

## EXAMPLE 1

500.000 I.U. salcatonin are dissolved in 900 ml distilled water containing 12.53 g citric acid, 12.37 g sodium citrate dihydrate, 1.5 g methyl p-hydroxybenzoate sodium salt, 0.23 g propyl p-hydroxybenzoate sodium salt, 1 g sodium edetate. Distilled water is added up to 1000 ml. After filtration on 0.2 $\mu$m filters, the solution is shared among glass bottles fitted with a 0.1 ml dispenser at a time, corresponding to 50 I.U. salcatonin.

## EXAMPLE 2

The process described in Example 1 is repeated using a double amount of salcatonin, i.e. 1.000.000 I.U. Each dispensing from the bottle corresponds to 100 I.U. salcatonin.

Table 1    T=20°C
Bottles 100 I.U.[*] (whole)

| | month 0 | month 2 | month 4 | month 6 | month 8 | month 10 | month 12 | month 16 | month 20 | month 24 |
|---|---|---|---|---|---|---|---|---|---|---|
| Appearance | corr | corr | corr | corr | corr | corr | corr | corr | corr | corr |
| Identification | corr | corr | corr | corr | corr | corr | corr | corr | corr | corr |
| Calcitonin content I.U.[*] | 100,58 | 100,46 | 100,24 | 100,16 | 100,03 | 99,47 | 98,35 | 97,30 | 95,21 | 93,48 |

[*] by dispensing

corr = corresponding

Table 2    T=20°C
Bottles 100 I.U.[*]
(partially dispensed)

| | month 0 | month 2 | month 4 | month 6 | month 8 | month 10 | month 12 | month 16 | month 20 | month 24 |
|---|---|---|---|---|---|---|---|---|---|---|
| Appearance | corr | corr | corr | corr | corr | corr | corr | corr | corr | corr |
| Identification | corr | corr | corr | corr | corr | corr | corr | corr | corr | corr |
| Calcitonin content I.U.[*] | 100,57 | 100,45 | 100,21 | 100,09 | 99,93 | 99,12 | 98,29 | 96,94 | 94,83 | 92,85 |

[*] by dispensing

corr = corresponding

EP 0 489 217 A1

Table 3    T=20
Bottles 100 I.U.*
without edetate (whole)

| | month 0 | month 2 | month 4 | month 6 | month 8 | month 10 | month 12 | month 16 | month 20 | month 24 |
|---|---|---|---|---|---|---|---|---|---|---|
| Appearance | corr | corr | corr | corr | corr | corr | corr | corr | corr | corr |
| Identification | corr | corr | corr | corr | corr | corr | corr | corr | corr | corr |
| Calcitonin content I.U.* | 100,51 | 98,3 | 93,6 | 91,9 | 90,1 | 89,2 | 87,2 | 84,4 | 82,2 | 80,1 |

*by dispensing
corr = corresponding

Table 4    T=20°C
Bottles 100 I.U.*
without edetate
(partially dispensed)

| | month 0 | month 2 | month 4 | month 6 | month 8 | month 10 | month 12 | month 16 | month 20 | month 24 |
|---|---|---|---|---|---|---|---|---|---|---|
| Appearance | corr | corr | corr | corr | corr | corr | corr | corr | corr | corr |
| Identification | corr | corr | corr | corr | corr | corr | corr | corr | corr | corr |
| Calcitonin content I.U.* | 100,52 | 96,2 | 91,8 | 90,0 | 88,2 | 86,4 | 84,2 | 81,9 | 80,1 | 79,6 |

*by dispensing
corr = corresponding

**Claims**

1. Liquid, stable in the air, pharmaceutical compositions, for intranasal administration, containing a) calcitonin dissolved in b) a citrate buffered aqueous solution at pH 4.0 characterized in that c) sodium

5

ethylenediaminetetraacetate is further contained.

2. Pharmaceutical compositions according to claim 1, further containing methyl and/or propyl p-hydroxybenzoates.

3. Pharmaceutical compositions according to claims 1 o 2, characterized in that calcitonin is contained in amounts from about 100 to about 5000 I.U. per millilitre.

4. Pharmaceutical compositions according to the preceding claims, each millilitre thereof containing:
from 250 to 2000 I.U. calcitonin;
from 6.27 to 18.8 mg citric acid;
from 6.19 to 18.56 mg sodium citrate dihydrate;
from 0.75 to 2.25 mg methyl p-hydroxybenzoate sodium salt;
from 0.11 to 0.34 mg propyl p-hydroxybenzoate sodium salt; and
from 0.5 to 1.5 mg ethylendiaminotetracetic acid tetrasodium salt.

5. Pharmaceutical compositions according to the preceding claims characterized in that calcitonin is salmon calcitonin (salcatonin).

**Claim for the following Contracting States : ES, GR**

1. A process for the preparation of liquid, stable in the air, pharmaceutical compositions containing calcitonin, for intranasal administration, characterized in dissolving from 250 to 2000 I.U. calcitonin per millilitre of final solution, into an aqueous solution containing from 6.27 to 18.8 mg citric acid; from 6.19 to 18.56 mg sodium citrate dihydrate; from 0.75 to 2.25 mg methyl p-hydroxybenzoate sodium salt; from 0.11 to 0.34 mg propyl p-hydroxybenzoate sodium salt; and from 0.5 to 1.5 mg ethylenediaminetetraacetic acid tetrasodium salt; for each millilitre of the final solution;
filtering the obtained solution and packing the same in suitable containers; being all the process steps carried out without inert gases protection.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 358 234 (RORER INTERNATIONAL) * Claims 1,3; page 2, lines 49-50; page 11, lines 23-27,33-39; page 12, lines 1-9; page 13, example 8; page 16, example 14 * | 1-5 | A 61 K 37/30 A 61 K 9/12 A 61 K 47/18 |
| E | EP-A-0 418 697 (ESSETI s.a.s. LABORATORIO CHIMICO FARMACO BIOLOGICO DI A. IEVOLI & C.) * Claims 1,4-6; page 3, lines 50-53; examples 1-4 * | 1-5 | |
| A | EP-A-0 399 781 (TEIJIN LTD) * Page 2, lines 6-8; page 4, lines 26-29 * | 1 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 05-07-1991 | VENTURA AMAT A. |